Europäisches Patentamt

European Patent Office

Office européen des brevets

(19)

(11) Veröffentlichungsnummer: **0 115 058**
**B1**

(12) **EUROPÄISCHE PATENTSCHRIFT**

(45) Veröffentlichungstag der Patentschrift:
27.01.88

(51) Int. Cl.⁴: **A 61 K 6/02**

(21) Anmeldenummer: 83113022.4

(22) Anmeldetag: 23.12.83

(54) Pulverförmiger Dentalwerkstoff, Verfahren zu seiner Herstellung und seine Verwendung.

(30) Priorität: 28.12.82 DE 3248357

(43) Veröffentlichungstag der Anmeldung:
08.08.84 Patentblatt 84/32

(45) Bekanntmachung des Hinweises auf die Patenterteilung:
27.01.88 Patentblatt 88/4

(84) Benannte Vertragsstaaten:
AT BE CH DE FR GB IT LI LU NL SE

(56) Entgegenhaltungen:
EP - A - 0 016 315
EP - A - 0 024 056
WO - A - 80/00409
US - A - 3 413 723
US - A - 4 209 434

(73) Patentinhaber: ESPE Stiftung & Co Produktions- und
Vertriebs KG, D-8031 Seefeld (DE)

(72) Erfinder: McLean, John W., Dr., 38, Devonshire Street,
London W1N ILD (GB)
Erfinder: Gasser, Oswald, Dr., Hartstrasse 13,
D-8031 Seefeld 1 (DE)

(74) Vertreter: Abitz, Walter, Dr.-Ing. et al, Abitz, Morf,
Gritschneder, Freiherr von Wittgenstein
Postfach 86 01 09, D-8000 München 86 (DE)

## Beschreibung

Die Erfindung betrifft einen pulverförmigen Dentalwerkstoff auf Grundlage von (a) Calciumaluminiumfluorosilicatglas, (b) für dentale Zwecke üblichen Edelmetallen, ihren Legierungen miteinander und/oder ihren Legierungen mit bis zu 40% unedlen Metallen, bezogen auf das Gewicht der Legierung, und gegebenenfalls (c) trockener Polycarbonsäure und/oder (d) chelatbildendem Mittel, dessen Herstellung und Verwendung.

Glasionomerzemente, die das Reaktionsprodukt aus einem Calciumaluminiumfluorosilikatglaspulver, einer wasserlöslichen Polycarbonsäure und Wasser darstellen, haben gegenüber anderen für Füllungszwecke verwendeten Dentalmaterialien (z.B. Composit-Kunststoffüllungen, Amalgam) den Vorteil, dass sie physiologisch sehr verträglich sind und mit der Zahnsubstanz eine chemische Verbindung eingehen, die einen perfekten Randschluss bewirkt. Nachteilig an den Glasionomerzementen sind ihre relativ hohe Sprödigkeit, die einen Einsatz im Molarenbereich und an Ecken und Kanten bisher nicht ermöglichen.

Aus der GB-A 20 28 855 sind metallhaltige Glasionomerzement-Pulver bekannt. Hierbei handelt es sich jedoch um blosse Gemische aus Zementpulver und Metallpulver, die keiner thermischen Behandlung unterworfen worden sind. Diese Gemische sind hinsichtlich ihrer Verschleissfestigkeit eher schlechter als reine Glasionomerzemente.

Aus der DE-A 30 42 008 ist ein Zahnfüllungsmaterial bekannt, das aus einer gerüstförmigen metallischen Komponente mit offenen Hohlräumen und einer nichtmetallischen Komponente, z.B. einem Glasionomerzement, als flüssigem oder plastischem Material besteht. Zur Herstellung wird die gerüstförmige metallische Komponente entweder mit der nicht-metallischen Komponente benetzt oder getränkt oder die gerüstförmige metallische Komponente wird in die nicht-metallische Komponente eingeknetet. Die gerüstförmige Metallkomponente kann durch Sintern von Metallpulvern hergestellt worden sein. Auch bei diesem Zahnfüllungsmaterial wird die Metallkomponente nur z.B. mit dem Glasionomerzement vermischt, ohne vorher mit einem Calciumaluminiumfluorosilicatglas versintert worden zu sein.

Aufgabe der Erfindung ist die Bereitstellung eines pulverförmigen Dentalwerkstoffes auf der Grundlage eines Glasionomerzement-Pulvers, der gegenüber reinem Glasionomerzement eine verbesserte Verschleissfestigkeit ergibt.

Diese Aufgage wird erfindungsgemäss durch die Schaffung eines pulverförmigen Dentalwerkstoffes auf der Grundlage von (a) Calciumaluminiumfluorosilicatglas, (b) für dentale Zwecke üblichen Edelmetallen, ihren Legierungen miteinander und/oder ihren Legierungen mit bis zu 40% unedlen Metallen, bezogen auf das Gewicht der Legierung, und gegebenenfalls (c) trockener Polycarbonsäure und/oder (d) chelatbildendem Mittel gelöst, der mindestens einen Teil des Bestandteils (a) als gesinterte Mischung mit dem Bestandteil (b) enthält.

Der neue pulverförmige Dentalwerkstoff wird dadurch hergestellt, dass man Pulver von (a) Calciumaluminiumfluorosilikatglas und (b) von für dentale Zwecke üblichen Edelmetallen, ihren Legierungen miteinander und/oder ihren Legierungen mit bis zu 40 Gewichtsprozent unedlen Metallen, bezogen auf das Gewicht der Legierung, vermischt, das erhaltene Gemisch, gegebenenfalls nach Verpressung zu Formkörpern, bei >600 °C sintert, das gesinterte Produkt zu einem Pulver vermahlt und gegebenenfalls das erhaltene Pulver mit (a) ungesintertem Calciumaluminiumfluorosilikatglas, (c) trockener Polycarbonsäure und/oder (d) chelatbildendem Mittel, jeweils in pulverförmiger Form, vermischt. Das erhaltene gesinterte Produkt kann gegebenenfalls mit (1) nicht gesintertem Calciumaluminiumfluorosilikatglas und/oder (2) mit trockener Polycarbonsäure und/oder (3) mit einem chelatbildenden Mittel, jeweils in pulverförmiger Form, vermischt werden.

Für die Verwendung wird der erfindungsgemässe pulverförmige Dentalwerkstoff im Falle (1) mit wässriger Polycarbonsäurelösung, die gegebenenfalls einen Chelatbildner enthält im Falle (2) mit gegebenenfalls chelatbildnerhaltigem Wasser, z.B. einer Weinsäurelösung, und im Falle (3) mit wässriger Polycarbonsäurelösung oder mit Wasser zu einem Zahnfüllmaterial vermischt, das sich durch hohe Verschleissfestigkeit auszeichnet und daher auch zur Modellierung von Kanten und Ecken im Backenzahnbereich geeignet ist. Dabei haftet der aus dem erfindungsgemässen Dentalwerkstoff hergestellte Zement an der Zahnsubstanz und hat eine gute physiologische Verträglichkeit.

Für den erfindungsgemässen Dentalwerkstoff verwendbare Calciumaluminiumfluorosilikatglas-Pulver sind in der DE-A 20 61 513 und der europäischen Patentschrift 23 013 beschrieben.

Die efindungsgemäss verwendeten Calciumaluminiumfluorosilikatglas-Pulver bestehen neben Sauerstoff vorzugsweise aus:

| Bestandteil | Berechnet als | Gew.-% |
|---|---|---|
| Si | $SiO_2$ | 20–60 |
| Al | $Al_2O_3$ | 10–50 |
| Ca | CaO | 1–40 |
| F | F | 1–40 |
| Na | $Na_2O$ | 0–10 |
| P | $P_2O_5$ | 0–10 |

und insgesamt 0–20 Gew.-%, berechnet als Oxide, an B, Bi, Zn, Mg, Sn, Ti, Zr, La oder anderen 3wertigen Lanthanoiden, K, W, Ge, sowie weiteren Zusätzen, welche die Eigenschaften nicht beeinträchtigen und physiologisch unbedenklich sind.

Vorteilhaft bestehen die Pulverteilchen aus:

| | |
|---|---|
| Si als $SiO_2$ | 25–50 Gew.-% |
| Al als $Al_2O_3$ | 10–40 Gew.-% |

| Ca als CaO | 10–35 Gew.-% |
| F | 5–30 Gew.-% |
| Na als $Na_2O$ | 0– 8 Gew.-% |
| P als $P_2O_5$ | 1–10 Gew.-% |

und 0–10 Gew.-% an $B_2O_3$, $Bi_2O_3$, ZnO, MgO, $SnO_2$, $TiO_2$, $ZrO_2$, $La_2O_3$ oder anderen Oxiden 3wertiger Lanthanoiden, $K_2O$, $WO_3$, $GeO_2$, sowie weiteren Zusätzen, welche die Eigenschaften nicht beeinträchtigen und physiologisch unbedenklich sind.

Besonders bevorzugt verwendete Pulver enthalten

| Si als $SiO_2$ | 25–45 Gew.-% |
| Al als $Al_2O_3$ | 20–40 Gew.-% |
| Ca als CaO | 10–30 Gew.-% |
| F | 10–30 Gew.-% |
| Na als $Na_2O$ | 1– 8 Gew.-% |
| P als $P_2O_5$ | 1–10 Gew.-% |

Beispiel der bevorzugt verwendeten Zusammensetzungen sind in der folgenden Tabelle I aufgeführt

Tabelle I

| | Gew.-% | | | |
| | A | B | C | D |
| --- | --- | --- | --- | --- |
| Si als $SiO_2$ | 35,0 | 27,6 | 29,0 | 45,4 |
| Al als $Al_2O_3$ | 30,4 | 26,0 | 25,1 | 35,0 |
| Ca als CaO | 14,9 | 28,8 | 24,6 | 10,1 |
| F | 17,7 | 17,0 | 23,0 | 10,4 |
| Na als $Na_2O$ | 2,7 | 2,1 | 2,2 | 6,9 |
| P als $P_2O_5$ | 6,9 | 8,3 | 5,8 | 2,4 |

Die erfindungsgemäss verwendeten Glaspulverteilchen können an der Oberfläche an Calcium so verarmt sein, dass der Quotient aus dem Atomverhältnis Si/Ca an der Oberfläche der Pulverteilchen und dem Atomverhältnis Si/Ca im Kernbereich mindestens 2,0, vorzugsweise mindestens 3,0 und besonders bevorzugt mindestens 4.0 beträgt (vg. Europäische Patentschrift 23 013). Besonders vorteilhaft werden diese Pulver als Zumischung zum gemahlenen, metallhaltigen Sinterprodukt verwendet.

Die erfindungsgemäss verwendeten Glaspulver weisen eine durchschnittliche Korngrösse (Gewichtsmittel) von wenigstens 1 µm und bevorzugt mindestens 3 µm auf. Die durchschnittliche Korngrösse (Gewichtsmittel) beträgt 1–20 µm, bevorzugt 3–15 µm und besonders bevorzugt 3–10 µm. Die Teilchen haben eine maximale Korngrösse von 150 µm, vorzugsweise 100 µm, besonders 60 µm. Für die Verwendung als dentaler Befestigungszement beträgt die maximale Teilchengrösse 25 µm, vorzugsweise 20 µm. Zur Erzielung guter mechanischer Eigenschaften ist in üblicher Weise eine nicht zu enge Korngrössenverteilung günstig, wie sie beispielsweise durch übliche Vermahlung und Absiebung der Grobanteile erreicht wird.

Die Glaspulver werden wie üblich durch Zusammenschmelzen der Ausgangs-Komponenten bei Temperaturen oberhalb 950 °C, Abschrecken und Mahlen gewonnen. Als Ausgangs-Komponenten können beispielsweise die in der DE-A 2 061 513 angegebenen Verbindungen in entsprechenden Mengenbereichen eingesetzt werden.

Die so erhaltenen Pulver werden dann gegebenenfalls einer Oberflächenbehandlung gemäss der Europäischen Patentschrift 23 013 unterzogen. Hierzu werden die Glaspulver mit Säure oberflächlich behandelt, vorzugsweise bei Raumtemperatur. Dabei werden saure Gruppen enthaltende Substanzen eingesetzt, z.B. Salzsäure, Schwefelsäure, Salpetersäure, Essigsäure, Propionsäure oder Perchlorsäure, die lösliche Calciumsalze bilden. Die Säuren werden in einer Konzentration von 0,01 bis 10 Gew.-%, vorzugsweise von 0,05 bis 3 Gew.-% eingesetzt.

Nach der entsprechenden Reaktionszeit werden die Pulver von der Lösung separiert und gründlich ausgewaschen, so dass sich praktisch keine löslichen Calciumsalze mehr auf der Oberfläche der Pulverteilchen befinden. Die oberflächlich an Calcium verarmten Pulverteilchen sind besonders als Zumischung zu den gemahlenen, metallhaltigen Sinterprodukten geeignet.

Als Edelmetallpulver sind die in der Dentaltechnik bekannten Edelmetalle, wie Gold, Platin, Palladium und Silber, geeignet, sowie ihre Legierungen untereinander und ihre Legierungen mit bis zu 40 Gew.-%, bezogen auf das Gewicht der Legierung, an unedlen Metallen, wie Kupfer, Zinn, Indium und Zink. Ein Beispiel für eine erfindungsgemäss verwendbare Legierung ist eine Legierung aus 65 Gew.-% Silber und 35 Gew.-% Zinn. Bevorzugt als Edelmetall werden Silber und Gold, sowie ihre Legierungen mit nicht mehr als 10 Gew.-% anderer Metalle.

Die durchschnittliche Korngrösse (Gewichtsmittel) der Edelmetallpulverkomponente (b) beträgt wenigstens 0,5 µm, bevorzugt wenigstens 1 µm und besonders bevorzugt mindestens 3 µm. Das Gewichtsmittel der Korngrössenverteilung liegt vorzugsweise zwischen 0,5 und 20 µm, insbesondere zwischen 1 und 10 µm. Die maximale Korngrösse der Metallpulver soll 60 µm, vorzugsweise 40 µm und ganz besonders 10 µm nicht überschreiten. Vorzugsweise weisen mindestens 90% der Teilchen der Edelmetallpulverkomponente (b) eine Korngrösse unterhalb 32 µm auf, besonders bevorzugt unterhalb 10 µm.

Der erfindungsgemässe pulverförmige Dentalwerkstoff kann auch trockene Polycarbonsäure enthalten; als solche können die zur Herstellung von Glasionomerzementpulver bekannten Polycarbonsäuren verwendet werden, z.B. Polymaleinsäure, Polyacrylsäure sowie Gemische davon, oder Copolymere, insbesondere Maleinsäure-Acrylsäure-Copolymere und/oder Acrylsäure-Itaconsäure-Copolymere.

Der erfindungsgemässe Dentalwerkstoff kann ausserdem ein chelatbildendes Mittel enthalten (vgl. DE-A 23 19 715). Vorzugsweise wird als Chelatbildner Weinsäure eingesetzt.

Zur Durchführung des erfindungsgemässen Verfahrens werden zunächst die pulverförmige Glaskomponente (a) und die pulverförmige Edelmetallkomponente (b) miteinander vermischt. Der Anteil der Edelmetallkomponente (b) an der Mischung von (a) und (b) kann 20 bis 80, vorzugsweise 40 bis 60 Vol.-% der Mischung von (a) + (b) betragen.

Die Glas/Metall-Pulvermischung kann direkt gesintert werden. Vorzugsweise wird sie aber zunächst zu Presslingen verarbeitet. Beispielsweise kann man in einer Tablettenpresse Presslinge herstellen. Vorzugsweise erfolgt das Verpressen unter hohem Druck, z.B. in einer hydraulischen Presse, wobei Drücke über 294 MPa (3000 kp/cm²) besonders vorteilhaft sind. Es ist günstig, während des Pressens die Presskammer zu evakuieren, beispielsweise auf einem Druck von weniger als 10 mbar.

Das Pulvergemisch oder die aus dem Gemisch hergestellten Presslinge werden dann bei über 600 °C gesintert. Vorzugsweise geschieht das Sintern im Vakuum, insbesondere unterhalb 10 mbar, ganz besonders unterhalb 5 mbar. Die Sintertemperatur wird vorzugsweise unterhalb des Schmelzpunkts der verwendeten Edelmetallkomponente (b) gehalten; wenn die Edelmetallkomponente aus Silber oder Gold besteht, ist eine Sintertemperatur von etwa 800 °C gut geeignet. Die Sinterzeit kann einige Minuten bis mehrere Stunden betragen, bei Presslingen je nach Grösse z.B. 10 Minuten bis 3 Stunden, vorzugsweise ca. 1 Stunde. Vor Entnahme des Sinterprodukts aus dem Glühofen ist es günstig, wenn es auf eine Temperatur unterhalb 600 °C abgekühlt ist.

Die erhaltenen Sinterkörper werden in üblicher Weise zu einem feinteiligen Pulver vermahlen. Die durchschnittliche Korngrösse (Gewichtsmittel) und die maximale Korngrösse des erfindungsgemässen pulverförmigen Dentalwerkstoffs sind die gleichen, wie sie oben für das für die Sinterung einzusetzende Calciumaluminiumfluorosilikatglas-Pulver beschrieben wurden.

Man kann das erhaltene Pulver aber auch mit dem oben beschriebenen ungesinterten Calciumaluminiumfluorosilikatglas-Pulver vermischen. Vorzugsweise setzt man 10 bis 50 Vol.-% ungesintertes Calciumaluminiumfluorosilikatglas-Pulver, bezogen auf das gesinterte Produkt, zu. Besonders geeignet sind dazu die an der Oberfläche an Calcium verarmten Pulver.

Man kann das gemahlene Sinterprodukt oder dessen Gemisch mit ungesintertem Glaspulver auch mit trockener, feinteiliger Polycarbonsäure im Gewichtsverhältnis 1:1 bis 5:1 vermischen.

Man kann einem dieser Gemische auch ein chelatbildendes Mittel in Pulverform zusetzen.

Die erfindungsgemässen pulverförmigen Dentalwerkstoffe werden in der für Glasionomerzementpulver üblichen Weise verwendet. Falls der erfindungsgemässe Dentalwerkstoff keine trockene Polycarbonsäure enthält, so wird er im Gewichtsverhältnis 2:1 bis 10:1, vorzugsweise 4:1 bis 8:1, mit einer wässrigen Polycarbonsäurelösung vermischt, in der gegebenenfalls ein Chelatbildner, z.B. Weinsäure, gelöst ist. Es sind die gleichen Polycarbonsäuren in Form ihrer wässrigen Lösung geeignet, die in feinteiliger trockener Form mit dem erfindungsgemässen, metallhaltigen Pulver vermischt werden können. Enthält der erfindungsgemässe Dentalwerkstoff bereits trockene Polycarbonsäure, so wird der Dentalwerkstoff mit Wasser, gegebenenfalls unter Zusatz eines Chelatbildners, zu einer zementartigen Konsistenz angemischt.

Mit dem erfindungsgemässen pulverförmigen Dentalwerkstoff hergestellte Zahnzemente ergeben Zahnfüllungen, die auch an Kanten und Ecken im Molarenbereich hervorragende Verschliessfestigkeit aufweisen, während Zahnfüllungen aus Glasionomerzementen ohne Metallgehalt oder mit eingemischtem Metallgehalt relativ rasch durch Kantenbruch und starke Abrasion zerstört werden.

Ausser für Füllungszwecke im Molarenbereich können die erfindungsgemässen Dentalwerkstoffe auch für die üblichen Füllungen verwendet werden, wenn sie nicht im direkt sichtbaren Gebiet des Gebisses liegen. Weiterhin kann der erfindungsgemässe Werkstoff zum Aufbau zerstörter Stümpfe vor der Überkronung eingesetzt werden. Wenn die maximale Korngrösse des Werkstoffs 20 μm nicht überschreitet, ist der erfindungsgemässe Dentalwerkstoff auch als Befestigungszement, z.B. für Kronen, Brücken, geeignet.

Vor allem bei Verwendung der grauen Metalle, wie Silber, kann der Farbeindruck des Füllungsmaterials durch Zugabe von bis zu 5% eines Weiss- oder Gelbpigments (z.B. Titandioxid, Zinkoxid) verbessert werden. Ein derartiger Werkstoff ist dann auch im Frontbereich auf der lingualen Seite für Füllungszwecke einsetzbar.

Beispiel 1

Goldhaltiges Sinterpulver

Ein Calciumaluminiumfluorosilikatglas-Pulver der Zusammensetzung A der Tabelle I (durchschnittliche Teilchengrösse 8 μm) wird im Volumenverhältnis 1:1 mit einem feinen Goldpulver (99% Gold, alle Teilchen < 5 μm) innig vermischt und unter einem Druck von 686 MPa (7000 kp/cm²) bei einem Vakuum von 4 mbar zu zylindrischen Presslingen geformt, die einen Durchmesser von 13 mm und eine Höhe von 5 mm haben.

Die Presslinge werden bei einem Vakuum von 4 mbar und einer Temperatur von 800 °C 1 Stunde lang erhitzt. Nach dem Erkalten des Ofens auf unter 600 °C werden die Presslinge entnommen und in einer Kugelmühle vermahlen. Anschliessend werden durch Sieben Teilchen von ≥ 40 μm entfernt.

Das erhaltene Pulver wird im Volumenverhältnis 4:1 mit dem oben beschriebenen ungesinterten Calciumaluminiumfluorosilikatglaspulver vermischt, das jedoch an der Oberfläche durch Behandlung mit 0,1% Salzsäure, wie in der europäischen Patentschrift 23013 beschrieben, verarmt wurde.

Der erhaltene Dentalwerkstoff wird im Gewichtsverhältnis 5:1 mit einer Lösung aus 37 g eines Copolymeren (1:1) aus Acrylsäure und Maleinsäure, 9 g Weinsäure und 54 g Wasser vermischt).

Die erhaltene Zementmischung hat eine Verarbeitungszeit von etwa 3 Minuten und eine Erhärtungszeit von etwa 8 Minuten.

Eine aus diesem Zement gelegte Molarenfüllung weist eine goldglänzende Oberfläche auf und zeigt nach 3 Jahren im Munde keine sichtbaren Verschleisserscheinungen. Oberflächenglanz und Farbe haben sich gegenüber dem Ausgangszustand sogar noch leicht verbessert.

Beispiel 2
Silberhaltiges Sinterpulver

17,5 Gewichtsteile eines Calciumaluminiumfluorosilikatglas-Pulvers der Zusammensetzung B der Tabelle I (durchschnittliche Teilchengrösse 6 µm) werden mit 82,5 Gewichtsteilen eines feinteiligen Silberpulvers (Silberpulver II F der Fa. Degussa; mittlere Teilchengrösse 3,5 µm, 99,9%ig) homogen vermischt und wie in Beispiel 1 beschrieben, durch Pressen, Sintern, Mahlen und Mischen mit dem gleichen unbehandelten Calciumaluminiumfluorosilikatglas-Pulver zu einem Dentalwerkstoff-Pulver verarbeitet.

Nach einer Verarbeitung, wie in Beispiel 1 angegeben, werden Zahnfüllungen im Molarenbereich gelegt. Die Füllungen zeigen einen metallisch grauen Glanz, der nach einer Beobachtungszeit von 2 Jahren nur leicht nachdunkelt; ein Verschleiss der Füllungen ist nicht zu erkennen.

Vergleichsversuch

Bei mehreren Probanden wurden im Molarenbereich Füllungen gelegt, deren Basis folgende Zementpulver waren:
a) erfindungsgemässer, goldhaltiger Dentalwerkstoff nach Beispiel 1;
b) erfindungsgemässer, silberhaltiger Dentalwerkstoff nach Beispiel 2;
c) Glasionomerzementpulver nach Beispiel 1 der europäischen Patentschrift 23013;
d) ein Pulver der Zusammensetzung entsprechend a), das jedoch keiner Sinterungsbehandlung unterworfen worden war.

Die Füllungen wurden nach einer Tragezeit von 2 Jahren begutachtet und zeigten folgende Ergebnisse:
a: intakte, goldglänzende Oberfläche, keine Verschleisserscheinungen;
b: intakte, glänzende, leicht nachgedunkelte Oberfläche, keine Verschleisserscheinungen;
c: leicht rauhe Oberfläche, im Bereich der Kanten Defekte durch Bruch;
d: rauhe Oberfläche mit deutlichem Abtrag, im Bereich der Kanten Brucherscheinungen.

## Patentansprüche

1. Pulverförmiger Dentalwerkstoff auf der Grundlage von (a) Calciumaluminiumfluorosilikatglas, (b) für dentale Zwecke üblichen Edelmetallen, ihren Legierungen miteinander und/oder ihren Legierungen mit bis zu 40% unedlen Metallen, bezogen auf das Gewicht der Legierung, und gegebenenfalls (c) trockener Polycarbonsäure und/oder (d) chelatbildendem Mittel, dadurch gekennzeichnet, dass er mindestens einen Teil des Bestandteils (a) als gesinterte Mischung mit dem Bestandteil (b) enthält.

2. Verfahren zur Herstellung des pulverförmigen Dentalwerkstoffs des Anspruchs 1, dadurch gekennzeichnet, dass man Pulver von (a) Calciumaluminiumfluorosilikatglas und (b) von für dentale Zwecke üblichen Edelmetallen, ihren Legierungen miteinander und/oder ihren Legierungen mit bis zu 40 Gewichtsprozent unedlen Metallen, bezogen auf das Gewicht der Legierung, vermischt, das erhaltene Gemisch, gegebenenfalls nach Verpressung zu Formkörpern, bei >600 °C sintert, das gesinterte Produkt zu einem Pulver vermahlt und gegebenenfalls das erhaltene Pulver mit (a) ungesintertem Calciumaluminiumfluorosilikatglas, (c) trockener Polycarbonsäure und/oder (d) chelatbildendem Mittel, jeweils in pulverförmiger Form, vermischt.

3. Verwendung des pulverförmigen Dentalwerkstoffs des Patentanspruchs 1 zur Herstellung von selbsthärtenden Glasionomerzementen.

## Claims

1. A powdery dental material based on (a) calcium aluminium fluorosilicate glass, (b) noble metals which are conventional for dental purposes, the alloys thereof with each other and/or the alloys thereof with up to 40% of base metals, with respect to the weight of the alloy, and optionally (c) dry polycarboxylic acid and/or (d) chelating agent, characterised in that it contains at least a portion of the component (a) in the form of a sintered mixture with the component (b).

2. A process for producing the powdery dental material according to Claim 1, characterised by mixing powder of (a) calcium aluminium fluorosilicate glass and (b) noble metals which are conventional for dental purposes, the alloys thereof with each other and/or the alloys thereof with up to 40% by weight of base metals, with respect to the weight of the alloy, sintering the resulting mixture, optionally after pressing it to provide shaped bodies, at >600 °C, crushing the sintered product to form a powder and optionally mixing the resulting powder with (a) unsintered calcium aluminium fluorosilicate glass, (c) dry polycarboxylic acid and/or (d) chelating agent, each in powder form.

3. Use of the powdery dental material of Claim 1 for the production of self-hardening glass ionomer cements.

## Revendications

1. Produit dentaire sous forme de poudre, à base (a) de verre au fluorosilicate de calcium et d'aluminium, (b) de métaux précieux, de leurs alliages les uns avec les autres et/ou de leurs alliages avec jusqu'à 40% de métaux communs, par rapport au poids de l'alliage, courants dans les applications dentaires, et éventuellement (c) d'un acide polycarboxylique sec et/ou (d) d'un agent chélatant, caractérisé en ce qu'il contient au moins une partie du constituant (a) sous la forme d'un mélange fritté avec le constituant (b).

2. Procédé pour la préparation du matériau dentaire sous forme de poudre selon la revendication 1, caractérisé en ce qu'on mélange une poudre (a) de verre au fluorosilicate de calcium et d'aluminium et (b) de métaux précieux, de leurs alliages les uns avec les autres et/ou de leurs alliages avec jusqu'à 40% en poids de métaux communs, par rapport au poids de l'alliage, courants dans les applications dentaires, qu'on fritte le mélange obtenu, éventuellement après compression pour obtenir des objets moulés, à une température > 600 °C, qu'on broie le produit fritté pour obtenir une poudre, et éventuellement qu'on mélange la poudre obtenue avec (a) un verre au fluorosilicate de calcium et d'aluminium non-fritté, (c) un acide polycarboxylique sec et/ou (d) un agent chélatant, chacun sous la forme d'une poudre.

3. Utilisation du produit dentaire sous forme de poudre selon la revendication 1 pour la préparation de ciments vitroionomères auto-durcissants.